# FASCICULE DE BREVET EUROPEEN

(11) **EP 1 796 614 B1**
(45) Date de publication et mention de la délivrance du brevet: **30.05.2018**
(21) Numéro de dépôt: 05805602.9
(22) Date de dépôt: 08.09.2005
(51) Int. Cl.: A61K 8/81, A61K 8/04, A61Q 5/06

(54) **DISPOSITIF AEROSOL CONTENANT AU MOINS UN POLYMERE DE TYPE AMPS ET AU MOINS UN POLYMERE FIXANT**
AEROSOLVORRICHTUNG MIT MINDESTENS EINEM AMPS-POLYMER UND MINDESTENS EINEM FIXIERENDEM POLYMER
AEROSOL DEVICE CONTAINING AT LEAST ONE AMPS POLYMER AND AT LEAST ONE FIXING POLYMER

(30) Priorité: 09.09.2004 FR 0452005; 21.10.2004 US 620424 P
(43) Date de publication de la demande: 20.06.2007
(73) Titulaire: L' Or Al, 75008 Paris (FR)
(72) Inventeur: GRINGORE, Charles, F-92600 Asnières-sur-Seine (FR); PATAUT, Françoise, F-75017 Paris (FR)
(74) Mandataire: Bourdeau, Françoise
(86) Numéro de dépôt international: PCT/FR2005/002237
(87) Numéro de publication internationale: WO 2006/030113

(56) Documents cités:
- EP-A- 1 325 732
- WO-A-00/49999
- FR-A- 2 701 394
- FR-A- 2 745 173
- FR-A- 2 779 643
- FR-A- 2 798 589

## Description

La présente invention concerne un dispositif aérosol contenant au moins un polymère de type AMPS et au moins un polymère fixant, ainsi qu'un procédé de coiffage utilisant un tel dispositif.

Les compositions de coiffage conditionnées sous forme de spray aérosol contiennent généralement une fraction importante d'alcool. Or, les produits cosmétiques à haute teneur en alcool font l'objet d'une surveillance particulière, notamment aux Etats Unis, suite à la sensibilisation récente de l'opinion publique aux problèmes écologiques résultant de l'émission de produits organiques volatils dans l'atmosphère.

Une solution permettant de diminuer la quantité d'alcool, voire de supprimer totalement l'alcool des formules, est le remplacement par une quantité équivalente d'eau. Cette introduction de quantités importantes d'eau dans les sprays aérosols destinés à la fixation des cheveux, tels que des laques, conduit toutefois à une modification importante indésirable de la forme de la chevelure et à une dégradation des propriétés cosmétiques. Par ailleurs, la plupart des agents propulseurs de type hydrocarbures sont incompatibles avec l'eau et leur utilisation dans des compositions à forte teneur en eau est de ce fait généralement impossible.

Les produits coiffants, sont habituellement utilisés pour apporter une tenue durable à la coiffure.

Pour cela, des polymères fixants sont introduits dans un milieu hydro alcoolique.

Le conditionnement sous forme d'aérosol permet une répartition régulière sur l'ensemble de la chevelure.

La fixation de la coiffure est alors quasi-immédiate et il n'est pas aisé, voire impossible de structurer la coiffure avec les mains car les cheveux sont figés instantanément.

Le problème posé par l'invention est de fournir une composition cosmétique capillaire fixant convenablement la chevelure, procurant de bonnes propriétés cosmétiques et séchant en un temps raisonnable, tout en se répartissant bien et en apportant une sensation de fraîcheur à l'application.

La présente invention a pour objet une composition telle que définie dans la revendication 1.

L'invention a également pour objet un procédé de coiffage consistant à vaporiser le mélange contenu dans ledit dispositif aérosol, soit directement sur les cheveux à mettre en forme, soit via les doigts, et à laisser sécher la chevelure ainsi traitée.

### COMPOSITION COIFFANTE

Tous les polymères fixants anioniques, non ioniques et leurs mélanges utilisés dans la technique peuvent être utilisés dans les compositions selon la présente demande.

Par polymère fixant, on entend au sens de la présente invention tout polymère susceptible d'apporter un maintien de la chevelure dans une forme donnée.

### POLYMERES FIXANTS

### POLYMERES FIXANTS ANIONIQUES

Les polymères fixants peuvent être solubles dans le milieu cosmétiquement acceptable ou insolubles dans ce même milieu et utilisés dans ce cas sous forme de dispersions de particules solides ou liquides de polymère (latex ou pseudolatex).

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono ou diacides carboxyliques insaturés tels que ceux répondant à la formule : 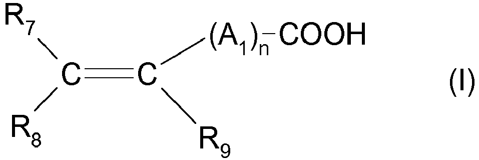 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL® E ou K par la société ALLIED COLLOID et ULTRAHOLD® par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques ;
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevet luxembourgeois n^{os} 75370 et 75371 ou proposés sous la dénomination QUADRAMER par la société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF ;
   On peut aussi citer les copolymères acide méthacrylique/ acide acrylique/acrylate d'éthyle/méthacrylate de méthyle en dispersion aqueuse, commercialisé sous la dénomination AMERHOLD® DR 25 par la société AMERCHOL ;
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch ;
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US nos 2 047 398, 2 723 248, 2 102 113, le brevet GB n° 839 805. Des produits commerciaux sont notamment ceux vendus sous les dénominations GANTREZ® AN ou ES par la société ISP ;
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.

   Ces polymères sont par exemple décrits dans les brevets français n^{os} 2 350 384 et 2 357 241 de la demanderesse ;
E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone ;
- les sels de l'acide polystyrène-sulfonique tels que les sels de sodium vendus par exemple sous la dénomination Flexan® 500 et Flexan® 130 par National Starch. Ces composés sont décrits dans le brevet FR 2 198 719 ;
- mentionnés dans le brevet US 4 128 631, et plus particulièrement l'acide polyacrylamidoéthylpropane-sulfonique vendu sous la dénomination COSMEDIA POLYMER HSP 1180 par HENKEL.

Comme autre polymère fixant anionique utilisable selon l'invention, on peut citer le polymère anionique séquencé branché vendu sous la dénomination Fixate G-100 par la société NOVEON.

Selon l'invention, les polymères fixants anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF et les copolymères acétate de vinyle/acide crotonique vendus sous la dénomination LUVISET CA 66 par la société BASF et les copolymères acétate de vinyle/acide crotonique greffés par du polyéthylèneglycol vendus sous la dénomination ARISTOFLEX® A par la société BASF, le polymère vendu sous la dénomination Fixate G-100 par la société NOVEON.

Parmi les polymères fixants anioniques cités ci-dessus, on préfère plus particulièrement utiliser dans le cadre de la présente invention, les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP, le polymère vendu sous la dénomination Fixate G-100 par la société NOVEON.

### POLYMERES FIXANTS NON IONIQUES

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH® LDM 6911, MOWILITH® DM 611 et MOWILITH® LDM 6070 proposés par la société HOECHST, les produits RHODOPAS® SD 215 et RHODOPAS® DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol® PLUS par la société BASF ; et
- les copolymères de vinyllactame tels qu'un copolymère poly(vinylpyrrolidone/vinyllactame) vendu sous le nom commercial Luvitec® VPC 55K65W par la société BASF, les copolymères poly(vinylpyrrolidone/acétate de vinyle) comme ceux commercialisés sous la dénomination PVPVA® S630L par la société ISP, Luviskol® VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol® VAP 343 par la société BASF.

Les groupes alkyles des polymères non ioniques mentionnés ci-dessus ont, de préférence, de 1 à 6 atomes de carbone.

Selon l'invention, on peut également utiliser des polymères fixants de type siliconés greffés, comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0 582 152 et WO 93/23009 et les brevets US 4,693,935, US 4,728,571 et US 4,972,037.

Ces polymères peuvent être amphotères, anioniques ou non ioniques, et ils sont de préférence anioniques ou non ioniques.

De tels polymères sont, par exemple, les copolymères susceptibles d'être obtenus par polymérisation radicalaire à partir du mélange de monomères formé :
a) de 50 à 90 % en poids d'acrylate de tertiobutyle,
b) de 0 à 40 % en poids d'acide acrylique,
c) de 5 à 40 % en poids d'un macromère siliconé de formule 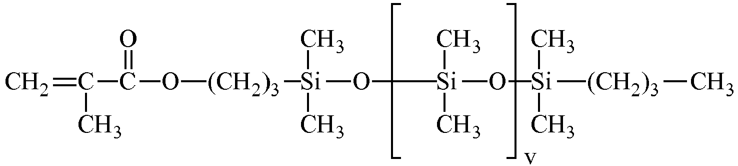
où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type poly(acide (méth)acrylique) et du type poly((méth)acrylate d'alkyle), et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Comme autre type de polymères fixants siliconés, on peut citer le produit Luviflex® Silk commercialisé par la société BASF.

La concentration en polymère(s) fixant(s) utilisée dans les compositions selon la présente invention est comprise entre 0,1 et 20%, de préférence entre 0,5 et 10% en poids par rapport au poids total de la composition.

### AMPS

Selon l'invention, la composition comprend au moins un polymère comportant au moins un monomère à insaturation éthylénique à groupement sulfonique, sous forme libre, ou partiellement ou totalement neutralisée choisi parmi
- l'acide poly-2-acrylamido-2-méthylpropane-sulfonique réticulé et partiellement ou totalement neutralisé ;
- les copolymères de l'acide 2-acrylamido-2-méthylpropane-sulfonique (AMPS) de formule (I) suivante, et d'au moins un monomère hydrophobe dont la partie hydrophobe comporte de 6 à 50 atomes de carbone, 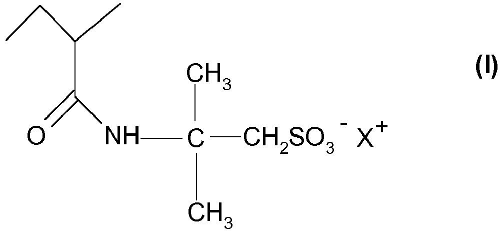 dans laquelle, X⁺ est un proton, ou un cation de métal alcalin, ou un cation alcalino-terreux, ou l'ion ammonium.

Les polymères sont représentés par des polymères comportant au moins un monomère à insaturation éthylénique à groupement sulfonique, sous forme libre ou partiellement ou totalement neutralisée, pouvant être modifiés par une chaîne grasse, ou des copolymères d'au moins un monomère à insaturation éthylénique à groupement sulfonique sous forme libre ou partiellement ou totalement neutralisée et d'au moins un monomère comportant une partie hydrophobe.

Les polymères peuvent être réticulés ou non-réticulés.

Les polymères réticulés sont plus particulièrement préférés.

De préférence, les polymères sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

### Monomères à insaturation éthylènique à groupement sulfonique

Ils sont choisis notamment parmi l'acide vinylsulfonique, l'acide styrènesulfonique, les acides (méth)acrylamido(C₁-C₂₂)alkylsulfoniques, les acides N-(C₁-C₂₂)alkyl(méth)acrylamido(C₁-C₂₂)alkylsulfoniques comme l'acide undécyl-acrylamido-méthane-sulfonique ainsi que leurs formes partiellement ou totalement neutralisées.

De préférence, on utilise les acides (méth)acrylamido(C₁-C₂₂) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique (AMPS), l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique, ainsi que leurs formes partiellement ou totalement neutralisées.

Plus particulièrement encore, on utilise l'acide 2-acrylamido-2-méthylpropane-sulfonique (AMPS) ainsi que ses formes partiellement ou totalement neutralisées.

### Monomères comportant une partie hydrophobe

Lorsque le polymère comporte un monomère comportant une partie hydrophobe, la partie hydrophobe, qui est une chaîne grasse, comporte de préférence de 6 à 50 atomes de carbone, plus préférentiellement de 6 à 22 atomes de carbone, plus préférentiellement encore de 6 à 18 atomes de carbone, et plus particulièrement de 12 à 18 atomes de carbone.

Parmi les polymères comportant au moins un monomère à insaturation éthylènique à groupement sulfonique, et ne comportant pas de monomère avec une partie hydrophobe, on peut citer notamment les homopolymères suivants :
- l'acide polyacrylamido-méthane-sulfonique,
- l'acide polyacrylamido-éthane-sulfonique,
- l'acide polyacrylamido-propane-sulfonique,
- l'acide poly-2-acrylamido-2-méthylpropane-sulfonique,
- l'acide poly-2-méthacrylamido-2-méthylpropane-sulfonique,
- l'acide poly-2-acrylamido-n-butane-sulfonique,
et leurs formes partiellement ou totalement neutralisées.

Des polymères de ce type, et notamment des acides poly-2-acrylamido-2-méthylpropane-sulfoniques, peuvent être réticulés et partiellement ou totalement neutralisés. Ils sont en général caractérisés par le fait qu'ils comprennent, distribués de façon aléatoire :
a) de 90 à 99,9% en poids de motifs de formule (I) suivante (AMPS): 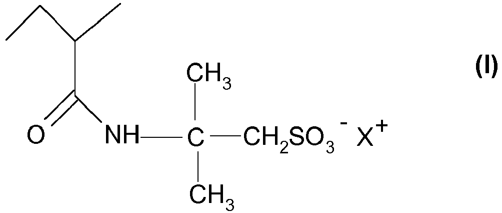 dans laquelle, X⁺ désigne un proton, ou un cation de métal alcalin, ou un cation équivalent de métal alcalino-terreux, ou le cation ammonium,
b) de 0,1 à 10% en poids d'au moins un motif réticulant ayant au moins deux double-liaisons oléfiniques,
les proportions en poids étant définies par rapport au poids total du polymère;
De préférence, l'acide poly-2-acrylamido-2-méthylpropane-sulfonique réticulé et neutralisé comporte de 98 à 99,5 % en poids de motifs de formule (I) et de 0,5 à 2 % en poids de motifs réticulants.

Les motifs réticulants ayant au moins deux doubles-liaisons oléfiniques sont choisis par exemple parmi le dipropylèneglycol-diallyléther, les polyglycol-diallyléthers, le triéthylèneglycol-divinyléther, l'hydroquinone-diallyl-éther, le tétra-allyl-oxyéthane ou d'autres allyl- ou vinyl-éthers d'alcools polyfonctionnels, le diacrylate de tétraéthylèneglycol, la triallylamine, le triméthylolpropane-diallyléther, le méthylène-bis-acrylamide ou le divinylbenzène.

Les motifs réticulants ayant au moins deux double-liaisons oléfiniques sont plus particulièrement encore choisis parmi ceux répondant à la formule générale (II) suivante : 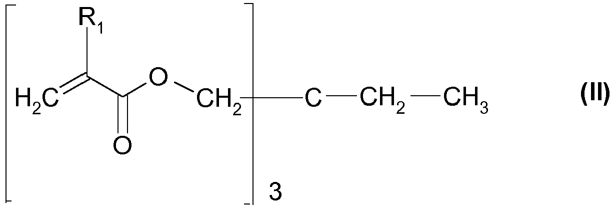 dans laquelle R₁ désigne un atome d'hydrogène ou un alkyle en C₁-C₄ et plus particulièrement méthyle (triméthylol propane triacrylate : TMPTA).

Les acides poly-2-acrylamido-2-méthylpropane-sulfoniques réticulés et partiellement ou totalement neutralisés sont généralement connus sous les appellations "Ammonium Polyacrylamido-2-methylpropanesulfonate" ou encore "Ammonium Polyacryloyldimethyltaurate" (dictionnaire I.N.C.I. ed.2000).

Ils sont par exemple décrits et préparés dans la demande de brevet DE-19625810.

Ils peuvent être obtenus selon le procédé de préparation connu comprenant les étapes suivantes :
(a) on disperse ou on dissout le monomère à insaturation éthylénique à groupement sulfonique sous forme libre dans une solution de tertio-butanol ou d'eau et de tertio-butanol ;
(b) on neutralise la solution ou la dispersion de monomère obtenue en (a) par une ou plusieurs bases minérales ou organiques, de préférence l'ammoniaque, dans une quantité permettant d'obtenir un taux de neutralisation des fonctions acides sulfoniques du polymère allant de 0 à 100% ;
(c) on ajoute à la solution ou dispersion obtenue en (b) le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

Un produit particulièrement préféré selon l'invention est celui vendu par la société CLARIANT sous la dénomination commerciale HOSTACERIN AMPS; c'est un acide poly-2-acrylamido-2-méthylpropane sulfonique réticulé et partiellement neutralisé par l'ammoniaque.

Les polymères peuvent aussi être choisis parmi les polymères statistiques d'AMPS modifiés par une chaîne grasse, par réaction avec une n-monoalkylamine ou une di-n-alkylamine en C₆-C₂₂, et tels que ceux décrits dans la demande de brevet WO00/31154 (faisant partie intégrante du contenu de la description). Ces polymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Polymères d'au moins un monomère à insaturation éthylénique à groupement sulfonique sous forme libre, ou partiellement ou totalement neutralisée, et d'au moins un monomère avec une partie hydrophobe.

De tels polymères ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Lorsqu'ils sont réticulés, les agents de réticulation peuvent être choisis parmi les composés à polyinsaturation oléfinique couramment utilisés pour la réticulation des polymères obtenus par polymérisation radicalaire.

On peut citer par exemple, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

On utilise plus particulièrement le méthylène-bis-acrylamide, le méthacrylate d'allyle ou le triméthylol propane triacrylate (TMPTA). Le taux de réticulation varie en général de 0,01 à 10% en mole et plus particulièrement de 0,2 à 2% en mole par rapport au polymère.

Parmi lesdits polymères, on préfère les copolymères d'AMPS de formule (I) et d'au moins un monomère hydrophobe ayant de 6 à 50 atomes de carbone et plus préférentiellement de 6 à 22 atomes de carbone et encore plus préférentiellement de 6 à 18 atomes de carbone et plus particulièrement 12 à 18 atomes de carbone.

Ces copolymères peuvent contenir en outre un ou plusieurs monomères, non hydrophobes, tels que les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

De tels copolymères sont décrits notamment dans la demande de brevet EP-A-750899, le brevet US 5089578 et dans les publications de Yotaro Morishima suivantes :
- « Self-assembling amphiphilic polyelectrolytes and their nanostructures - Chinese Journal of Polymer Science Vol. 18, N°40, (2000), 323-336. »
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
- « Solution properties of miscelle networks formed by non-ionic moieties covalently bound to an polyelectrolyte : salt effects on rheological behavior - Langmuir, 2000, Vol.16, N°12, 5324-5332 » ;
- « Stimuli responsive amphiphilic copolymers of sodium 2-(acrylamido)-2-methylpropanesulfonate and associative macromonomers - Polym. Preprint, Div. Polym. Chem. 1999, 40(2), 220-221».

Dans ces copolymères particuliers d'AMPS, les monomères comportant une partie hydrophobe sont choisis de préférence parmi les acrylates ou les acrylamides de formule (III) suivante : 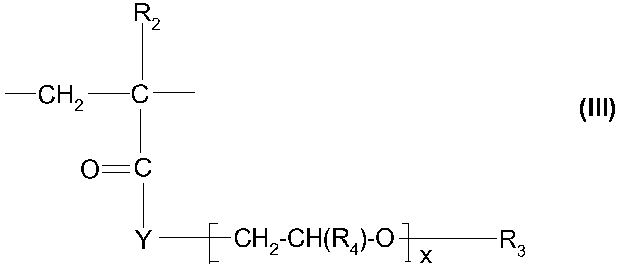 dans laquelle R₂ et R₄, identiques ou différents, désignent un atome d'hydrogène ou un radical alkyle linéaire ou ramifié en C₁-C₆ (de préférence méthyle) ; Y désigne O ou NH ; R₃ désigne un radical hydrocarboné hydrophobe comportant au moins de 6 à 50 atomes de carbone et plus préférentiellement de 6 à 22 atomes de carbone et encore plus préférentiellement de 6 à 18 atomes de carbone et plus particulièrement de 12 à 18 atomes de carbone ; x désigne un nombre de moles d'oxyde d'alkylène et varie de 0 à 100.

Le radical R₃ est choisi de préférence parmi les radicaux alkyles en C₆-C₁₈ linéaires (par exemple n-hexyle, n-octyle, n-décyle, n-hexadécyle, n-dodécyle), ramifiés ou cycliques (par exemple cyclododécane (C₁₂) ou adamantane (C₁₀)) ; les radicaux alkylperfluorés en C₆-C₁₈ (par exemple le groupement de formule - (CH₂)₂-(CF₂)₉-CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Selon une forme particulièrement préférée, le monomère de formule (III) comporte au moins un motif oxyde d'alkylène (x ≥1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100 et plus préférentiellement de 3 à 50 et encore plus préférentiellement de 7 à 25.

Parmi ces polymères, on peut citer :
- les copolymères réticulés ou non réticulés, neutralisés ou non, comportant de 15 à 60% en poids de motifs AMPS et de 40 à 85% en poids de motifs
   (C₈-C₁₆)alkyl(méth)acrylamide ou de motifs (C₈-C₁₆)alkyl(méth)acrylate par rapport au polymère, tels que ceux décrits dans la demande EP-A750 899 ;
- les terpolymères comportant de 10 à 90% en mole de motifs acrylamide, de 0,1 à 10% en mole de motifs AMPS et de 5 à 80% en mole de
   motifs n-(C₆-C₁₈)alkylacrylamide, tels que ceux décrits dans le brevet US- 5089578.

On peut également citer les copolymères non-réticulés et réticulés d'AMPS partiellement ou totalement neutralisé et de méthacrylate de dodécyle ainsi que les copolymères non-réticulés et réticulés d'AMPS partiellement ou totalement neutralisé et de n-dodécylméthacrylamide, tels que ceux décrits dans les articles de Morishima cités ci-dessus.

On cite plus particulièrement encore les copolymères constitués de motifs AMPS de formule (I) décrite ci-dessus et de motifs de formule (IV) suivante : 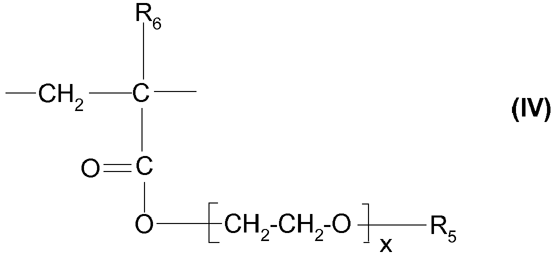 dans laquelle x désigne un nombre entier variant de 3 à 100, de préférence de 5 à 80 et
plus préférentiellement de 7 à 25 ; R₆ a la même signification que celle de R₂ indiquée ci-dessus dans la formule (III) et R₅ désigne un alkyle linéaire ou ramifié en C₆-C₂₂ et plus préférentiellement en C₁₀-C₂₂.

Les polymères particulièrement préférés sont ceux pour lesquels x = 25 , R₆ désigne méthyle et R₅ représente n-dodécyle ; ils sont décrits dans les articles de Morishima mentionnés ci-dessus.

Les polymères pour lesquels X⁺ désigne sodium ou ammonium sont plus particulièrement préférés.

Ces polymères préférés peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le ABAH (2,2-azobis-[2-amidinopropane] hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ils sont notamment obtenus par polymérisation radicalaire en milieu tert-butanol dans lequel ils précipitent.

En utilisant la polymérisation par précipitation dans le tert-butanol, il est possible d'obtenir une distribution de la taille des particules du polymère particulièrement favorable pour ses utilisations.

La distribution de la taille des particules du polymère peut être déterminée par exemple par diffraction laser ou analyse d'image.

Une distribution intéressante pour de type de polymère et déterminée par analyse d'image est la suivante: 60,2% inférieur à 423 microns, 52,0% inférieur à 212 microns, 26,6% inférieur à 106 microns, 2,6% inférieur à 45 microns et 26,6% supérieur à 850 microns.

La réaction peut être conduite à une température comprise entre 0 et 150°C, de préférence entre 10 et 100°C , soit à pression atmosphérique, soit sous pression réduite. Elle peut aussi être réalisée sous atmosphère inerte, et de préférence sous azote.

Selon ce procédé, on a notamment polymérisé l'acide 2-acrylamido-2-méthylpropane-sulfonique (AMPS) ou l'un de ses sels de sodium ou d'ammonium, avec un ester de l'acide (méth)acrylique et,
- d'un alcool en C₁₀-C₁₈ oxyéthyléné par 8 moles d'oxyde d'éthylène (GENAPOL® C-080 de la société HOECHST/CLARIANT),
- d'un alcool oxo en C₁₁ oxyéthyléné par 8 moles d'oxyde d'éthylène (GENAPOL® UD-080 de la société HOECHST/CLARIANT),
- d'un alcool oxo en C₁₁ oxyéthyléné par 7 moles d'oxyde d'éthylène (GENAPOL® UD-070 de la société HOECHST/CLARIANT),
- d'un alcool en C₁₂-C₁₄ oxyéthyléné par 7 moles d'oxyde d'éthylène (GENAPOL® LA-070 de la société HOECHST/CLARIANT),
- d'un alcool en C₁₂-C₁₄ oxyéthyléné par 9 moles d'oxyde d'éthylène (GENAPOL® LA-090 de la société HOECHST/CLARIANT),
- d'un alcool en C₁₂-C₁₄ oxyéthyléné par 11 moles d'oxyde d'éthylène (GENAPOL® LA-110 de la société HOECHST/CLARIANT),
- d'un alcool en C₁₆-C₁₈ oxyéthyléné par 8 moles d'oxyde d'éthylène (GENAPOL® T-080 de la société HOECHST/CLARIANT),
- d'un alcool en C₁₆-C₁₈ oxyéthyléné par 15 moles d'oxyde d'éthylène (GENAPOL® T-150 de la société HOECHST/CLARIANT),
- d'un alcool en C₁₆-C₁₈ oxyéthyléné par 11 moles d'oxyde d'éthylène (GENAPOL® T-110 de la société HOECHST/CLARIANT),
- d'un alcool en C₁₆-C₁₈ oxyéthyléné par 20 moles d'oxyde d'éthylène (GENAPOL® T-200 de la société HOECHST/CLARIANT),
- d'un alcool en C₁₆-C₁₈ oxyéthyléné par 25 moles d'oxyde d'éthylène (GENAPOL® T-250 de la société HOECHST/CLARIANT),
- d'un alcool en C₁₈-C₂₂ oxyéthyléné par 25 moles d'oxyde d'éthylène et/ou d'un alcool iso C₁₆-C₁₈ oxyéthyléné par 25 moles d'oxyde d'éthylène.

La concentration molaire en % des motifs de formule (I) et des motifs de formule (IV) dans les polymères peut varier entre 0,1 et 99,9% en moles.

De préférence pour les polymères les plus hydrophobes, la proportion molaire en motifs de formule (III) ou (IV) varie de 50,1 à 99,9%, plus particulièrement de 70 à 95% et encore plus particulièrement de 80 à 90%.

De préférence pour les polymères peu hydrophobes, la proportion molaire en motifs de formule (III) ou (IV) varie de 0,1 à 50%, plus particulièrement de 5 à 25% et encore plus particulièrement de 10 à 20%.

La distribution des monomères dans les polymères peut être, par exemple, alternée, bloc (y compris multibloc) ou quelconque.

On préfère que les polymères aient des chaînes pendantes sensibles à la chaleur et dont la solution aqueuse présente une viscosité qui, au delà d'une certaine température seuil, augmente, ou demeure pratiquement constante quand la température augmente.

Plus particulièrement encore, on préfère les polymères dont la solution aqueuse présente une viscosité qui est faible en dessous d'une première température seuil et qui, au dessus de cette première température seuil croît vers un maximum quand la température augmente, et qui, au dessus d'une seconde température seuil décroît à nouveau quand la température augmente. Dans cet optique, on préfère que la viscosité des solutions de polymère en dessous de la première température seuil soit de 5 à 50%, en particulier de 10 à 30% de la viscosité maximum à la seconde température seuil.

Ces polymères, de préférence, conduisent dans l'eau à un phénomène de démixion par chauffage se traduisant par des courbes présentant, en fonction de la température et de la concentration, un minimum appelé LCST (Lower Critical Solution Temperature).

Les viscosités (mesurées à 25°C au viscosimètre Brookfield aiguille 7) des solutions aqueuses à 1% vont de préférence de 20 000 mPa.s à 100 000 mPa.s et plus particulièrement de 60 000 mPa.s à 70 000 mPa.s.

Les polymères AMPS conformes à l'invention peuvent être présents dans les compositions selon l'invention dans des concentrations allant d'environ 0,01 à 30% en poids, de préférence de 0,1 à 20%, et plus particulièrement de 0,5 à 15% par rapport au poids total de la composition.

### AEROSOL

L'agent propulseur peut être n'importe quel gaz liquéfiable utilisé habituellement dans des dispositifs aérosol tels que le diméthyléther, les alcanes en C₃₋₅, le 1,1-difluoroéthane, les mélanges de diméthyléther et d'alcanes en C₃₋₅, et les mélanges de 1,1-difluoroéthane et de diméthyléther et/ou d'alcanes en C₃₋₅. On préfère tout particulièrement utiliser comme agent propulseur le diméthyl éther. L'agent propulseur est de préférence présent à plus de 20 %, et de préférence entre 20 et 70 %, et encore plus préférentiellement entre 20 et 50 % du poids total du mélange (agent propulseur (a) + composition coiffante (b)) contenu dans le dispositif aérosol.

Bien que les compositions de coiffage contenues dans le dispositif aérosol de la présente invention soient de préférence exemptes d'alcools inférieurs, elles peuvent contenir jusqu'à 50 % et de préférence jusqu'à 25% en poids, rapporté au poids total du mélange contenu dans le dispositif aérosol, d'un ou de plusieurs alcools inférieurs tels que l'éthanol ou l'isopropanol.

La composition coiffante selon l'invention peut contenir en outre au moins un adjuvant choisi parmi les silicones sous forme soluble, dispersée, micro-dispersée, les agents tensio-actifs non-ioniques, anioniques, cationiques et amphotères, les polymères additionnels autres que les polymères fixants et les polymères d'AMPS utilisés dans les compositions selon l'invention, les céramides et pseudo-céramides, les vitamines et pro-vitamines dont le panthénol, les huiles végétales, animales, minérales et synthétiques, les cires autres que les céramides et pseudo-céramides, les filtres solaires hydrosolubles et liposolubles, siliconés ou non siliconés, les particules solides telles que par exemple les pigments minéraux et organiques, colorés ou non colorés, les agents nacrants et opacifiants, les paillettes, particules actives, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les agents de pénétration, les parfums et les agents conservateurs.

L'homme de métier veillera à choisir les éventuels additifs et leur quantité de manière à ce qu'ils ne nuisent pas aux propriétés des compositions selon la présente invention.

Ces additifs sont présents dans la composition selon l'invention en une quantité allant de 0 à 20 % en poids par rapport au poids total de la composition.

Dans les compositions de coiffage contenues dans le dispositif aérosol, l'eau représente de 20 à 80 % en poids, rapporté au poids total du mélange contenu dans le dispositif aérosol.

Bien entendu l'homme du métier veillera à choisir ce ou ces éventuels additifs complémentaires et/ou leurs quantités de manière à ce que les propriétés avantageuses intrinsèques à l'invention ne soient pas altérées par la ou les adjonctions envisagées.

Avec cette association particulière, on obtient une mise en forme de la coiffure par un travail aux doigts sans obtenir le durcissement et le figeage instantanés classiquement rencontrés avec les produits de fixation. On peut par ailleurs obtenir sur les doigts ou sur la main un effet de crépitement durable conférant à la composition un caractère rhéologique original, apportant une sensation de fraîcheur à l'application. En effet, cette composition présente l'avantage de bien s'étaler sur les cheveux. L'application peut se faire aussi bien sur cheveux humides que secs et ceci directement ou via les doigts. Pour une application sur cheveux humides un séchage à l'air libre ou avec chaleur peut être réalisé.

Le résultat coiffure est souple et naturel.

L'exemple de formulation illustre la présente invention sans cependant la limiter.

### Exemple :

-----Aérosol comprenant en g/100g :
PVP / VA (BASF) : 3.25
Acide polyacrylamido méthyl propane sulfonique neutralisé partiellement à l'ammoniaque et hautement réticulé proposé sous la dénomination Aristoflex HMS par la société CLARIANT :0.97
Eau : 39.65
Alcool : 21.13
DME : 35.00

Cette composition « crépite » dans la main et induit un effet de fraîcheur. Elle permet après séchage un maintien durable de la coiffure.

## Revendications

1. Composition conditionnée dans un dispositif aérosol, contenant
(a) au moins un agent propulseur et
(b) une composition coiffante comprenant au moins (i) un polymère fixant anionique ou non ionique non polyuréthane et (ii) au moins un polymère comportant au moins un monomère à insaturation éthylénique à groupement sulfonique, sous forme libre, ou partiellement ou totalement neutralisée choisi parmi
- l'acide poly-2-acrylamido-2-méthylpropane-sulfonique réticulé et partiellement ou totalement neutralisé ;
- les copolymères de l'acide 2-acrylamido-2-méthylpropane-sulfonique (AMPS) de formule (I) suivante, et d'au moins un monomère hydrophobe dont la partie hydrophobe comporte de 6 à 50 atomes de carbone, 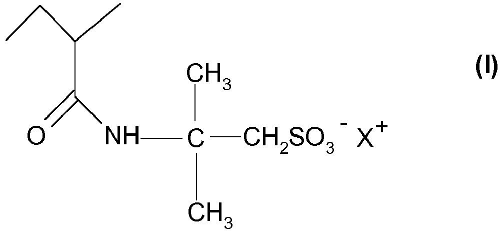 dans laquelle, X⁺ est un proton, ou un cation de métal alcalin, ou un cation alcalino-terreux, ou l'ion ammonium,
l'agent propulseur (a) représentant de 20 % à 70% du poids total du mélange contenu dans le dispositif aérosol.

2. Composition selon la revendication 1, **caractérisée par le fait que** la partie hydrophobe du polymère comporte de 6 à 22 atomes de carbone.

3. Composition selon l'une quelconque des revendications précédentes, **caractérisée par le fait que** les polymères sont neutralisés partiellement ou totalement par une base minérale ou organique.

4. Composition selon l'une quelconque des revendications précédentes, **caractérisée par le fait que** les polymères sont réticulés ou non-réticulés.

5. Composition selon la revendication 4, **caractérisée par le fait que** les polymères sont réticulés.

6. Composition selon l'une quelconque des revendications précédentes, **caractérisée par le fait que** le monomère hydrophobe est choisi parmi les acrylates ou les acrylamides de formule (III) suivante : 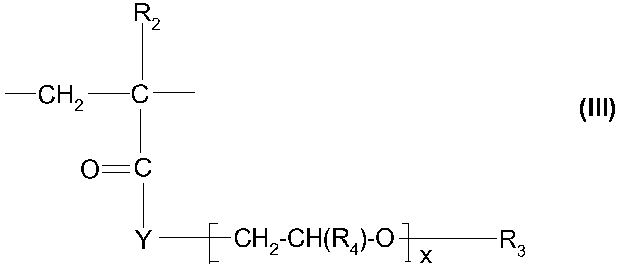 dans laquelle R₂ et R₄, identiques ou différents, désignent un atome d'hydrogène ou un radical alkyle linéaire ou ramifié en C₁-C₆ (de préférence méthyle) ; Y désigne O ou NH ; R₃ désigne un radical hydrocarboné hydrophobe comportant au moins de 6 à 50 atomes de carbone, de préférence de 6 à 22 atomes de carbone, encore plus préférentiellement de 6 à 18 atomes de carbone et plus particulièrement de 12 à 18 atomes de carbone ; x désigne un nombre de moles d'oxyde d'alkylène et varie de 0 à 100.

7. Composition selon la revendication 6, **caractérisée par le fait que** le radical hydrophobe R₃ est choisi parmi les radicaux alkyles en C₆-C₁₈, linéaires, ramifiés ou cycliques ; les radicaux alkylperfluorés en C₆-C₁₈ ; le radical cholestéryle ou un ester de cholestérol ; les groupes polycycliques aromatiques.

8. Composition selon l'une quelconque des revendications précédentes, **caractérisée par le fait que** le polymère est choisi parmi les copolymères de l'acide 2-acrylamido-2-méthylpropane-sulfonique (AMPS) de formule (I), et d'au moins un monomère hydrophobe de formule (IV) suivante : 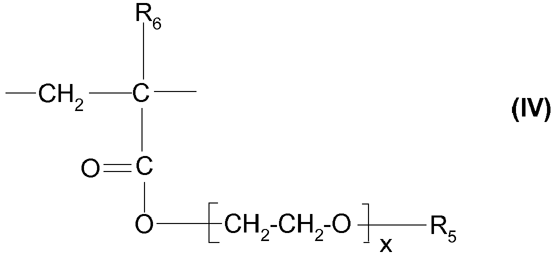 dans laquelle, x désigne un nombre entier variant de 3 à 100, de préférence de 5 à 80 et plus préférentiellement de 7 à 25 ; R₆ a la même signification que celle de R₂ indiquée ci-dessus dans la formule (III) et R₅ désigne un alkyle linéaire ou ramifié en C₆-C₂₂ et plus préférentiellement en C₁₀-C₂₂.

9. Composition selon l'une quelconque des revendications précédentes **caractérisée par le fait que** le polymère est un copolymère de l'acide 2-acrylamido-2-méthylpropane-sulfonique (AMPS) de formule (I), et de n.dodécylacrylamide [formule (III), où x=0, R₂=H, Y=NH, R₃ = n.C₁₂], neutralisé.

10. Composition selon l'une quelconque des revendications précédentes **caractérisée par le fait que** le polymère est un copolymère réticulé de l'acide 2-acrylamido-2-méthylpropane-sulfonique (AMPS) de formule (I) neutralisés par NH₃ et de motifs de formule (IV) dans laquelle R₆ désigne l'hydrogène ou un radical méthyle, R₅=C₁₆-C₁₈ et x=25.

11. Composition selon l'une quelconque des revendications précédentes, **caractérisée par le fait que** le polymère (ii) représente de 0,01 à 30%, de préférence de 0,1 à 20% et plus particulièrement de 0,5 à 15% en poids du poids total de la composition.

12. Composition selon l'une quelconque des revendications précédentes, **caractérisée en ce que** le polymère fixant anionique est choisi parmi les homopolymères ou copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les homopolymères ou copolymères à groupes sulfoniques, et les polymères siliconés greffés anioniques.

13. Composition selon l'une quelconque des revendications précédentes, **caractérisée en ce que** le polymère fixant non ionique est choisi parmi les polyalkyloxazolines, les homopolymères et copolymères d'acétate de vinyle, les homopolymères et copolymères d'esters acryliques, les copolymères d'acrylonitrile, les homopolymères et copolymères de styrène, les polyamides, les homopolymères de vinyllactame différents des homopolymères de vinylpyrrolidone, les copolymères de vinyllactame, et les polymères siliconés greffés non ioniques.

14. Composition cosmétique selon l'une des revendications précédentes telle que la concentration en polymère(s) fixant(s) (i) est comprise entre 0,1 et 20%, de préférence entre 0,5 et 10% en poids par rapport au poids total de la composition.

15. Composition selon l'une quelconque des revendications précédentes, **caractérisée par le fait que** l'eau représente de 20 à 80 % en poids, rapporté au poids total du mélange contenu dans le dispositif aérosol.

16. Composition selon l'une quelconque des revendications précédentes, **caractérisée par le fait que** l'agent propulseur (a) représente de 20 % à 50% du poids total du mélange contenu dans le dispositif aérosol.

17. Composition selon l'une quelconque des revendications précédentes, **caractérisée par le fait que** l'agent propulseur est choisi parmi les alcanes C3 C5, le difluoroéthane, le diméthyl-éther ou leurs mélanges.

18. Composition selon la revendication 17 **caractérisée en ce que** l'agent propulseur est le diméthyléther.

19. Composition selon l'une quelconque des revendications précédentes, **caractérisée par le fait qu'**il s'agit d'une laque pour cheveux.

20. Composition selon l'une quelconque des revendications précédentes, **caractérisée par le fait qu'**elle contient en outre au moins un adjuvant choisi parmi les silicones sous forme soluble, dispersée, micro-dispersée, les agents tensio-actifs non-ioniques, anioniques, cationiques et amphotères, les polymères additionnels autres que les polymères fixants ou les polyAMPS utilisés dans les compositions selon l'invention, les céramides et pseudo-céramides, les vitamines et pro-vitamines dont le panthénol, les huiles végétales, animales, minérales et synthétiques, les cires autres que les céramides et pseudo-céramides, les filtres solaires hydrosolubles et liposolubles, siliconés ou non siliconés, les particules solides telles que par exemple les pigments minéraux et organiques, colorés ou non colorés, les agents nacrants et opacifiants, les paillettes, particules actives, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les agents de pénétration, les parfums et les agents conservateurs.

21. Procédé de coiffage des cheveux consistant à vaporiser le mélange contenu dans le dispositif aérosol selon l'une quelconque des revendications précédentes directement sur les cheveux à mettre en forme ou via les doigts et à laisser sécher la chevelure ainsi traitée.

## Patentansprüche

1. In einer Aerosolvorrichtung abgepackte Zusammensetzung, enthaltend
(a) mindestens ein Treibmittel und
(b) eine Haarfrisierzusammensetzung, umfassend mindestens (i) ein anionisches oder nichtionisches fixierendes Nicht-Polyurethan-Polymer und (ii) mindestens ein Polymer, das mindestens ein ethylenisch ungesättigtes Monomer mit einer Sulfonsäuregruppe in freier oder teilweiser oder neutralisierter Form umfasst, das aus
- vernetzter und teilweise oder vollständig neutralisierter Poly-2-acrylamido-2-methylpropan-sulfonsäure,
- Copolymeren von 2-Acrylamido-2-methylpropan-sulfonsäure (AMPS) der folgenden Formel (I) und mindestens einen hydrophoben Monomer, dessen hydrophober Teil 6 bis 50 Kohlenstoffatome umfasst, 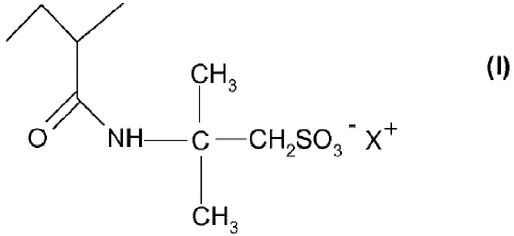 wobei X⁺ für ein Proton oder ein Alkalimetallkation oder ein Erdalkalikation oder ein Ammoniumion steht,
ausgewählt ist,
wobei das Treibmittel (a) 20 bis 70 Gew.-% des Gesamtgewichts der in der Aerosolvorrichtung enthaltenen Mischung ausmacht.

2. Zusammensetzung nach Anspruch 1, **dadurch gekennzeichnet, dass** der hydrophobe Teil des Polymers 6 bis 22 Kohlenstoffatome umfasst.

3. Zusammensetzung nach einem der vorhergehenden Ansprüche, **dadurch gekennzeichnet, dass** die Polymere teilweise oder vollständig mit einer anorganischen oder organischen Base neutralisiert sind.

4. Zusammensetzung nach einem der vorhergehenden Ansprüche, **dadurch gekennzeichnet, dass** die Polymere vernetzt oder unvernetzt sind.

5. Zusammensetzung nach Anspruch 4, **dadurch gekennzeichnet, dass** die Polymere vernetzt sind.

6. Zusammensetzung nach einem der vorhergehenden Ansprüche, **dadurch gekennzeichnet, dass** das hydrophobe Monomer aus Acrylaten oder Acrylamiden der folgenden Formel (III) ausgewählt sind: 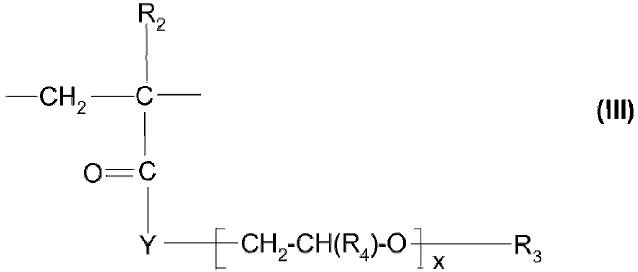 wobei R₂ und R₄, die gleich oder verschieden sind, für ein Wasserstoffatom oder einen linearen oder verzweigten C₁-C₆-Alkylrest (vorzugsweise Methyl) stehen; Y für O oder NH steht; R₃ für einen hydrophoben Kohlenwasserstoffrest mit mindestens 6 bis 50 Kohlenstoffatomen, vorzugsweise 6 bis 22 Kohlenstoffatomen und noch weiter bevorzugt 6 bis 18 Kohlenstoffatomen und weiter bevorzugt 12 bis 18 Kohlenstoffatomen steht; x für eine Zahl der Mole von Alkylenoxid steht und von 0 bis 100 variiert.

7. Zusammensetzung nach Anspruch 6, **dadurch gekennzeichnet, dass** der hydrophobe Rest R₃ aus linearen, verzweigten oder cyclischen C₆-C₁₈-Alkylresten; C₆-C₁₈-Perfluoralkylresten; dem Cholesterylrest oder einem Cholesterinester und polycyclischen aromatischen Gruppen ausgewählt ist.

8. Zusammensetzung nach einem der vorhergehenden Ansprüche, **dadurch gekennzeichnet, dass** das Polymer aus Copolymeren von 2-Acrylamido-2-methyl-propansulfonsäure (AMPS) der Formel (I) und mindestens einem hydrophoben Monomer der folgenden Formel (IV): 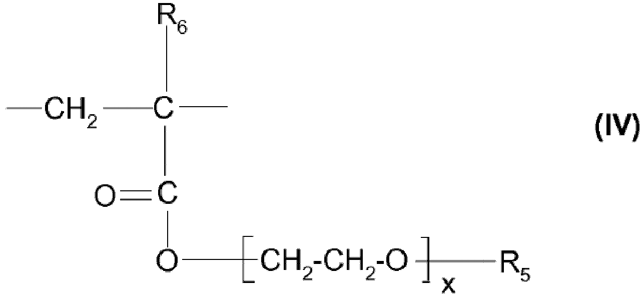 wobei x für eine ganze Zahl von 3 bis 100, vorzugsweise von 5 bis 80 und weiter bevorzugt von 7 bis 25 steht; R₆ die gleiche Bedeutung wie die oben in der Formel (III) angegebene Bedeutung von R₂ hat und R₅ für ein lineares oder verzweigtes C₆-C₂₂- und weiter bevorzugt C₁₀-C₂₂-Alkyl steht, ausgewählt ist.

9. Zusammensetzung nach einem der vorhergehenden Ansprüche, **dadurch gekennzeichnet, dass** es sich bei dem Polymer um ein neutralisiertes Copolymer von 2-Acrylamido-2-methylpropansulfonsäure (AMPS) der Formel (I) und von n-Dodecylacrylamid [Formel (III), wobei x = 0, R₂ = H, Y = NH, R₃ = n-C₁₂] handelt.

10. Zusammensetzung nach einem der vorhergehenden Ansprüche, **dadurch gekennzeichnet, dass** es sich bei dem Polymer um ein vernetztes Copolymer von mit NH₃ neutralisierter 2-Acrylamido-2-methyl-propansulfonsäure (AMPS) der Formel (I) und Einheiten der Formel (IV), in der R₆ für Wasserstoff oder einen Methylrest steht, R₅ = C₁₆-C₁₈ und x = 25, handelt.

11. Zusammensetzung nach einem der vorhergehenden Ansprüche, **dadurch gekennzeichnet, dass** das Polymer (ii) 0,01 bis 30 Gew.-%, vorzugsweise 0,1 bis 20 Gew.-% und weiter bevorzugt 0,5 bis 15 Gew.-% des Gesamtgewichts der Zusammensetzung ausmacht.

12. Zusammensetzung nach einem der vorhergehenden Ansprüche, **dadurch gekennzeichnet, dass** das anionische fixierende Polymer aus Homopolymeren oder Copolymeren von Acrylsäure und Methacrylsäure oder Salzen davon, Copolymeren von Crotonsäure, Copolymeren von einfach ungesättigten C₄-C₈-Carbonsäuren oder -Carbonsäureanhydriden, Polyacrylamiden mit Carboxylatgruppen, Homopolymeren oder Copolymeren mit Sulfonsäuregruppen und anionischen Silikonpfropfpolymeren ausgewählt ist.

13. Zusammensetzung nach einem der vorhergehenden Ansprüche, **dadurch gekennzeichnet, dass** das nichtionische fixierende Polymer aus Polyalkyloxazolinen, Homopolymeren und Copolymeren von Vinylacetat, Homopolymeren und Copolymeren von Acrylsäureestern, Copolymeren von Acrylnitril, Homopolymeren und Copolymeren von Styrol, Polyamiden, Homopolymeren von Vinyllactam, die von Homopolymeren von Vinylpyrrolidon verschieden sind, Copolymeren von Vinyllactam und nichtionischen Silikonpfropfpolymeren ausgewählt ist.

14. Kosmetische Zusammensetzung nach einem der vorhergehenden Ansprüche, wobei die Konzentration von fixierendem Polymer bzw. fixierenden Polymeren (i) zwischen 0,1 und 20 Gew.-% und vorzugsweise zwischen 0,5 und 10 Gew.-%, bezogen auf das Gesamtgewicht der Zusammensetzung, liegt.

15. Zusammensetzung nach einem der vorhergehenden Ansprüche, **dadurch gekennzeichnet, dass** Wasser 20 bis 80 Gew.-%, bezogen auf das Gesamtgewicht der in der Aerosolvorrichtung enthaltenen Mischung, ausmacht.

16. Zusammensetzung nach einem der vorhergehenden Ansprüche, **dadurch gekennzeichnet, dass** das Treibmittel (a) 20 bis 50 Gew.-% des Gesamtgewichts der in der Aerosolvorrichtung enthaltenen Mischung ausmacht.

17. Zusammensetzung nach einem der vorhergehenden Ansprüche, **dadurch gekennzeichnet, dass** das Treibmittel aus C3-C5-Alkanen, Difluorethan, Dimethylether oder Mischungen davon ausgewählt ist.

18. Zusammensetzung nach Anspruch 17, **dadurch gekennzeichnet, dass** es sich bei dem Treibmittel um Dimethylether handelt.

19. Zusammensetzung nach einem der vorhergehenden Ansprüche, **dadurch gekennzeichnet, dass** es sich um einen Haarlack handelt.

20. Zusammensetzung nach einem der vorhergehenden Ansprüche, **dadurch gekennzeichnet, dass** sie außerdem mindestens ein Adjuvans enthält, das aus Silikonen in löslicher, dispergierter und mikrodispergierter Form, nichtionischen, anionischen, kationischen und amphoteren Tensiden, zusätzlichen Polymeren, die von den in den erfindungsgemäßen Zusammensetzungen verwendeten fixierenden Polymeren bzw. PolyAMPS verschieden sind, Ceramiden und Pseudoceramiden, Vitaminen und Provitaminen, darunter Panthenol, pflanzlichen, tierischen, mineralischen und synthetischen Ölen, Wachsen, die von Ceramiden und Pseudoceramiden verschieden sind, silikonhaltigen oder nicht silikonhaltigen, wasserlöslichen und fettlöslichen Lichtschutzmitteln, festen Teilchen wie beispielsweise farbigen oder farblosen anorganischen und organischen Pigmenten, Perlglanz- und Trübungsmitteln, Pailletten, Wirkstoffteilchen, Farbmitteln, Sequestriermitteln, Weichmachern, Lösungsvermittlern, Ansäuerungsmitteln, Alkalinisierungsmitteln, Neutralisationsmitteln, anorganischen oder organischen Verdickungsmitteln, Antioxidantien, Hydroxysäuren, Penetriermitteln, Duftstoffen und Konservierungsmitteln ausgewählt ist.

21. Haarfrisierverfahren, das darin besteht, dass man die in der Aerosolvorrichtung enthaltene Mischung nach einem der vorhergehenden Ansprüche direkt oder über die Finger auf den in Form zu bringenden Haaren zerstäubt und das so behandelte Haar trocknen lässt.

## Claims

1. Composition packaged in an aerosol device, comprising
(a) at least one propellant and
(b) a styling composition comprising at least (i) one nonpolyurethane anionic or nonionic fixing polymer and (ii) at least one polymer comprising at least one ethylenically unsaturated monomer comprising a sulfo group in the free or partially or completely neutralized form,
- crosslinked and partially or completely neutralized poly(2-acrylamido-2-methylpropanesulfonic acid);
- copolymers of 2-acrylamido-2-methylpropanesulfonic acid (AMPS) of following formula (I) and of at least one hydrophobic monomer, the hydrophobic part of which comprises from 6 to 50 carbon atoms, 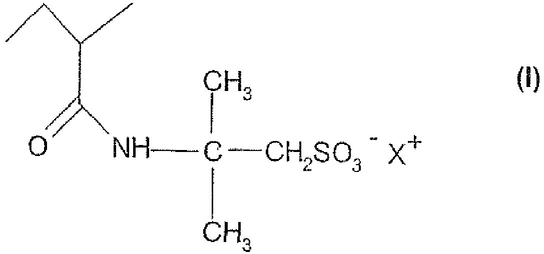 in which X⁺ is a proton or an alkali metal cation or an alkaline earth cation or the ammonium ion,
the propellant (a) representing from 20% to 70% of the total weight of the mixture present in the aerosol device.

2. Composition according to Claim 1, **characterized in that** the hydrophobic part of the polymer comprises from 6 to 22 carbon atoms.

3. Composition according to either one of the preceding claims, **characterized in that** the polymers are partially or completely neutralized by an inorganic or organic base.

4. Composition according to any one of the preceding claims, **characterized in that** the polymers are crosslinked or noncrosslinked.

5. Composition according to Claim 4, **characterized in that** the polymers are crosslinked.

6. Composition according to any one of the preceding claims, **characterized in that** the hydrophobic monomer is chosen from the acrylates or the acrylamides of following formula (III): 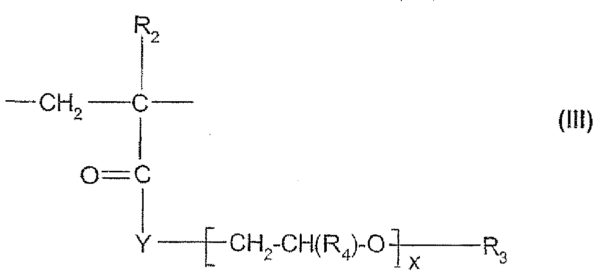 in which R₂ and R₄, which are identical or different, denote a hydrogen atom or a linear or branched C₁-C₆ alkyl radical (preferably methyl); Y denotes O or NH; R₃ denotes a hydrophobic hydrocarbon radical comprising at least from 6 to 50 carbon atoms, preferably from 6 to 22 carbon atoms, more preferably still from 6 to 18 carbon atoms and more particularly from 12 to 18 carbon atoms; x denotes a number of moles of alkylene oxide and varies from 0 to 100.

7. Composition according to Claim 6, **characterized in that** the hydrophobic radical R₃ is chosen from C₆-C₁₈ alkyl radicals which are linear, branched or cyclic; perfluorinated C₆-C₁₈ alkyl radicals; the cholesteryl radical or a cholesterol ester; or polycyclic aromatic groups.

8. Composition according to any one of the preceding claims, **characterized in that** the polymer is chosen from copolymers of 2-acrylamido-2-methylpropanesulfonic acid (AMPS) of formula (I) and of at least one hydrophobic monomer of following formula (IV): 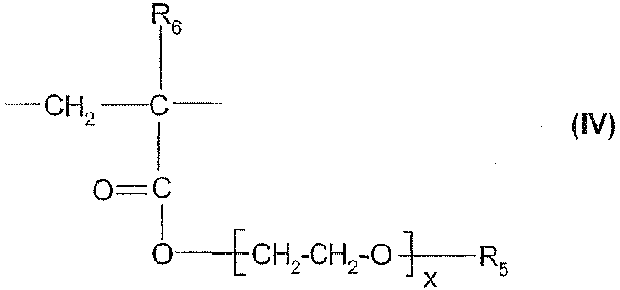 in which x denotes an integer varying from 3 to 100, preferably from 5 to 80 and more preferably from 7 to 25; R₆ has the same meaning as that of R₂ indicated above in the formula (III) and R₅ denotes a linear or branched C₆-C₂₂ and more preferably C₁₀-C₂₂ alkyl.

9. Composition according to any one of the preceding claims, **characterized in that** the polymer is a neutralized copolymer of 2-acrylamido-2-methylpropanesulfonic acid (AMPS) of formula (I) and of n-dodecylacrylamide [formula (III), where x = 0, R₂ = H, Y = NH, R₃ = n-C₁₂].

10. Composition according to any one of the preceding claims, **characterized in that** the polymer is a crosslinked copolymer of 2-acrylamido-2-methylpropanesulfonic acid (AMPS) of formula (I) neutralized by NH₃ and of units of formula (IV) in which R₆ denotes hydrogen or a methyl radical, R₅ = C₁₆-C₁₈ and x = 25.

11. Composition according to any one of the preceding claims, **characterized in that** the polymer (ii) represents from 0.01 to 30% by weight, preferably from 0.1 to 20% by weight and more particularly from 0.5 to 15% by weight, of the total weight of the composition.

12. Composition according to any one of the preceding claims, **characterized in that** the anionic fixing polymer is chosen from homopolymers or copolymers of acrylic or methacrylic acid or their salts, copolymers of crotonic acid, copolymers of C₄-C₈ monounsaturated carboxylic acids or anhydrides, polyacrylamides comprising carboxylate groups, homopolymers or copolymers comprising sulfo groups, and anionic grafted silicone polymers.

13. Composition according to any one of the preceding claims, **characterized in that** the nonionic fixing polymer is chosen from polyalkyloxazolines, vinyl acetate homopolymers and copolymers, acrylic ester homopolymers and copolymers, acrylonitrile copolymers, styrene homopolymers and copolymers, polyamides, vinyllactam homopolymers other than vinylpyrrolidone homopolymers, vinyllactam copolymers and nonionic grafted silicone polymers.

14. Cosmetic composition according to one of the preceding claims, such that the concentration of fixing polymer(s) (i) is between 0.1 and 20% by weight, preferably between 0.5 and 10% by weight, with respect to the total weight of the composition.

15. Composition according to any one of the preceding claims, **characterized in that** the water represents from 20 to 80% by weight, with respect to the total weight of the mixture present in the aerosol device.

16. Composition according to any one of the preceding claims, **characterized in that** the propellant (a) represents from 20% to 50% of the total weight of the mixture present in the aerosol device.

17. Composition according to any one of the preceding claims, **characterized in that** the propellant is chosen from C₃-C₅ alkanes, difluoroethane, dimethyl ether or their mixtures.

18. Composition according to Claim 17, **characterized in that** the propellant is dimethyl ether.

19. Composition according to any one of the preceding claims, **characterized in that** it is a hair lacquer.

20. Composition according to any one of the preceding claims, **characterized in that** it additionally comprises at least one adjuvant chosen from silicones in the soluble, dispersed or microdispersed form, nonionic, anionic, cationic and amphoteric surface-active agents, additional polymers other than the fixing polymers or the polyAMPSs used in the compositions according to the invention, ceramides and pseudoceramides, vitamins and provitamins, including panthenol, vegetable, animal, mineral and synthetic oils, waxes other than ceramides and pseudoceramides, water-soluble and fat-soluble sunscreens which may or may not comprise a silicone portion, solid particles, such as, for example, colored or colorless inorganic and organic pigments, pearlescent and opacifying agents, glitter, active particles, dyes, sequestering agents, plasticizing agents, solubilizing agents, acidifying agents, basifying agents, neutralizing agents, inorganic and organic thickening agents, antioxidants, hydroxy acids, penetrating agents, fragrances and preservatives.

21. Method for styling the hair, which consists in vaporizing the mixture present in the aerosol device according to any one of the preceding claims directly over the hair to be shaped or via the fingers and in allowing the hair thus treated to dry.
